# FASCICULE DE BREVET EUROPEEN

(11) **EP 3 903 795 B1**
(45) Date de publication et mention de la délivrance du brevet: **03.01.2024**
(21) Numéro de dépôt: 21180715.1
(22) Date de dépôt: 23.11.2017
(51) Int. Cl.: A61P 25/00, A61L 27/00

(54) **UNITE DE TISSU NEURAL ET UTILISATION D'UNE TELLE UNITE POUR L' IMPLANTATION DANS LE SYSTEME NERVEUX D'UN MAMMIFERE**
NERVENGEWEBEEINHEIT UND VERWENDUNG SOLCH EINER EINHEIT ZUR IMPLANTATION IM NERVENSYSTEM EINES SÄUGERS
NEURAL TISSUE UNIT AND USE OF SUCH A UNIT FOR IMPLANTATION IN THE NERVOUS SYSTEM OF A MAMMAL

(30) Priorité: 23.11.2016 FR 1661378
(43) Date de publication de la demande: 03.11.2021
(62) Demande divisionnaire de: 17817779.6
(73) Titulaire: UNIVERSITE DE BORDEAUX, 33000 Bordeaux (FR); Centre national de la recherche scientifique, 75016 Paris (FR); Institut d'Optique Théorique et Appliquée, 91127 Palaiseau Cedex (FR)
(72) Inventeur: FEYEUX, Maxime, 33400 TALENCE (FR); ALESSANDRI, Kevin, 33000 BORDEAUX (FR); NASSOY, Pierre, 33000 BORDEAUX (FR); COGNET, Laurent, 33000 BORDEAUX (FR); BENAZZOUZ, Abdelhamid, 33600 PESSAC (FR); BEZARD, Erwan, 33000 BORDEAUX (FR)
(74) Mandataire: Aquinov

(56) Documents cités:
- SONJA KRIKS ET AL: "'Floor plate-derived dopamine neurons from hESCs efficiently engraft in animal models of PD.'", NATURE, vol. 480, no. 7378, 6 novembre 2011 (2011-11-06), pages 547-551, XP055271235, ISSN: 0028-0836, DOI: 10.1038/nature10648
- KEVIN ALESSANDRI ET AL: "A 3D printed microfluidic device for production of functionalized hydrogel microcapsules for culture and differentiation of human Neuronal Stem Cells (hNSC)", LAB ON A CHIP, vol. 16, no. 9, 1 janvier 2016 (2016-01-01), pages 1593-1604, XP055393107, ISSN: 1473-0197, DOI: 10.1039/C6LC00133E
- MITSUE KOMATSU ET AL: "Maturation of human iPS cell-derived dopamine neuron precursors in alginate-Ca 2+ hydrogel", BIOCHIMICA ET BIOPHYSICA ACTA (BBA) - GENERAL SUBJECTS, vol. 1850, no. 9, 1 septembre 2015 (2015-09-01), pages 1669-1675, XP055405283, AMSTERDAM, NL ISSN: 0304-4165, DOI: 10.1016/j.bbagen.2015.04.011
- DATABASE BIOSIS [Online] BIOSCIENCES INFORMATION SERVICE, PHILADELPHIA, PA, US; September 2015 (2015-09), KOMATSU MITSUE ET AL: "Maturation of human iPS cell-derived dopamine neuron precursors in alginate-Ca2+ hydrogel", Database accession no. PREV201500623876 & BIOCHIMICA ET BIOPHYSICA ACTA, vol. 1850, no. 9, September 2015 (2015-09) , pages 1669-1675, ISSN: 0304-4165(print), DOI: 10.1016/J.BBAGEN.2015.04.011

## Description

L'invention est définie dans les revendications et a trait à une unité de tissu neural, comprenant une pluralité de cellules neuronales post-mitotiques, et éventuellement des cellules gliales, organisées en réseau à trois dimensions (3D) dans une matrice extracellulaire, et à son utilisation comme unité à implanter dans le système nerveux d'un mammifère. Une telle unité de tissu neural peut notamment être utilisée pour greffer des cellules neuronales présentant un phénotype d'intérêt dans le système nerveux d'un sujet, afin de traiter une maladie neurodégénérative.

Depuis plusieurs décennies, les maladies neurodégénératives constituent un défi majeur pour la société, tant en termes de compréhension des phénomènes biologiques impliqués qu'en termes de traitement. En effet, de plus en plus de personnes sont touchées par ces maladies, dont les conséquences pour le patient comme pour l'entourage sont dramatiques.

Le terme générique de « maladie neurodégénérative » regroupe un très grand nombre de maladies, caractérisées principalement par une mort neuronale plus rapide que lors d'un vieillissement normal, parmi lesquelles la maladie d'Alzheimer, la maladie de Parkinson, la chorée de Huntington, la sclérose latérale amyotrophique, etc. Le système nerveux peut être diversement touché, entraînant une variété de symptômes dont des troubles de la motricité, de l'équilibre, du comportement, et/ou de la cognition.

A l'heure actuelle, les causes et les mécanismes des maladies neurodégénératives sont souvent mal connus, rendant leur traitement d'autant plus complexe. Il n'existe pour l'instant aucun traitement probant permettant de guérir de ces maladies.

Depuis quelques années, des axes de développement ont porté sur la thérapie cellulaire intracérébrale et plus particulièrement sur la greffe de neurones dans les zones lésées du cerveau de sujets atteints de maladies neurodégénératives. Le but de ces greffes est de remplacer durablement les neurones détruits par la maladie. Ainsi, des greffes localisées de cellules ou de tissus embryonnaires ont été pratiquées avec succès chez plusieurs patients atteints de la maladie de Parkinson. Cependant, cette approche implique l'utilisation de tissus issus de foetus avortés, ce qui pose des problèmes éthiques et logistiques. Aussi, de nombreux laboratoires se sont tournés vers les cellules souches induites pluripotentes humaines (hIPS) comme source alternative possible de cellules humaines de qualité. La différenciation efficace des hIPS en neurones dotés du phénotype nécessaire pour remplacer les neurones perdus par les patients est un domaine de recherche très actif. Cependant, les neurones sont des cellules très fragiles.

L'injection d'une suspension de neurones aboutit généralement à la mort de plus de 98% desdits neurones par anoïkose. De manière alternative, il a été envisagé de greffer des progéniteurs neuronaux, connus pour être moins fragiles que les neurones. Cette solution ne donne pas non plus satisfaction. En effet, la différenciation cellulaire des progéniteurs neuronaux après implantation n'est pas contrôlée, et il n'est pas certain que les cellules implantées se différencient convenablement et/ou dans le type neuronal souhaité.

MITSUE KOMATSU ET AL ("Maturation of human iPS cell-derived dopamine neuron precursors in alginate-Ca 2+ hydrogel", BIOCHIMICA ET BIOPHYSICA ACTA (BBA) - GENERAL SUBJECTS, vol. 1850, no. 9, 1 septembre 2015), décrit l'encapsulation (hydrogel d'alginate) d'agrégats de précurseurs de neurones dopaminergiques et leur différenciation en neurones dopaminergiques. La couche d'hydrogel est ensuite éliminée et l'amas de neurones dopaminergiques est implanté dans le cerveau d'un rat.

Il existe donc un besoin en un système d'implantation de cellules neuronales permettant d'améliorer la survie des cellules implantées, afin notamment de remplacer durablement chez un patient atteint d'une maladie neurodégénérative les neurones altérés.

### Résumé de l'invention

En travaillant sur la culture de cellules en trois dimensions (3D), les inventeurs ont découvert qu'il est possible d'améliorer le taux de survie à la manipulation de cellules particulièrement fragiles telles que les neurones, en les cultivant et en les manipulant non pas sous forme de suspensions cellulaires, mais sous forme de réseaux cellulaires tridimensionnels. Ils ont ainsi mis au point une unité de tissu neural comprenant des cellules neuronales présentant un phénotype d'intérêt, qui peut être injectée dans le système nerveux d'un patient, afin d'y intégrer lesdites cellules neuronales avec un taux de survie bien supérieur à celui obtenu lors de l'injection d'une suspension de cellules neuronales. L'unité de cellules neurales mise au point comprend des cellules neurales pré-organisées en un réseau 3D et noyées dans une matrice extracellulaire, ce qui favorise l'intégration et la survie des cellules dans le système nerveux après injection. En effet, les cellules neurales sont transférées dans le système nerveux hôte en maintenant l'intégrité de leur environnement local, notamment les interactions entre cellules et avec la matrice extracellulaire développées au sein de l'unité de tissu neural. En outre, l'unité de tissu neural selon l'invention comprend des cellules neuronales post-mitotiques dont le degré de différenciation peut être parfaitement maîtrisé, limitant les risques d'introduire des types cellulaires non désirés, responsables notamment du développement de tératomes et d'excroissances tumorales. De manière intéressante, il a été constaté que l'unité de tissu neural selon l'invention est capable de projeter des axones au sein du tissu hôte, favorisant une bonne intégration des cellules dans ledit tissu hôte, et produisant un bénéfice fonctionnel certain. L'invention a donc pour objet une unité de tissu neural formant un amas de cellules neurales pour son utilisation en thérapie cellulaire pour une implantation dans le système nerveux d'un mammifère humain ou non humain, ladite unité de tissu neural étant capable de projeter des axones au sein du tissu hôte, dans laquelle ladite unité de tissu neural contient des cellules neuronales post-mitotiques différenciées immuno-compatibles avec le mammifère destiné à recevoir l'unité de tissu neural et dont le phénotype correspond au phénotype des cellules défaillantes à remplacer, organisées en trois dimensions et noyées dans une couche de matrice extracellulaire, ladite unité étant obtenue à partir d'un microcompartiment cellulaire comprenant une capsule en hydrogel entourant une unique unité de tissu neural, et ladite capsule en hydrogel étant au moins partiellement éliminée avant implantation de ladite unité de tissu neural.

Ainsi, l'invention a pour objet une unité de tissu neural formant un amas de cellules neurales pour son utilisation en thérapie cellulaire pour le traitement d'une maladie neurodégénérative par implantation dans le système nerveux d'un mammifère humain ou non humain présentant une maladie neurodégénérative, d'au moins une unité de tissu neural, capable de projeter des axones au sein du tissu hôte, contenant des cellules neuronales post-mitotiques différenciées immuno-compatibles avec le mammifère destiné à recevoir l'unité de tissu neural et dont le phénotype correspond au phénotype des cellules défaillantes à remplacer, organisées en trois dimensions et noyées dans une couche de matrice extracellulaire, ladite unité étant obtenue à partir d'un microcompartiment cellulaire comprenant une capsule en hydrogel entourant ladite unité de tissu neural, et ladite capsule en hydrogel étant au moins partiellement éliminée avant implantation de l'unité de tissu neural.

Notamment, l'invention a pour objet une unité de tissu neural formant un amas de cellules neurales pour son utilisation en thérapie cellulaire pour le traitement de la maladie de Parkinson, par implantation dans le système nerveux d'un mammifère humain ou non humain présentant la maladie de Parkinson, d'au moins une unité neurale capable de projeter des axones au sein du tissu hôte, contenant des cellules neuronales post-mitotiques différenciées en neurones dopaminergiques immuno-compatibles avec le mammifère destiné à recevoir l'unité de tissu neural, organisées en trois dimensions et noyées dans une couche de matrice extracellulaire, ladite unité étant obtenue à partir d'un microcompartiment cellulaire comprenant une capsule en hydrogel entourant ladite unité de tissu neural, et ladite capsule en hydrogel étant au moins partiellement éliminée avant implantation de l'unité de tissu neural.

L'invention a également pour objet une méthode de traitement de la maladie de Parkinson, selon laquelle on implante dans le système nerveux d'un patient présentant la maladie de Parkinson au moins une unité de tissu neural formant un amas de cellules neurales, contenant des cellules neuronales post-mitotiques différenciées en neurones dopaminergiques immuno-compatibles avec le mammifère destiné à recevoir l'unité de tissu neural, organisées en trois dimensions et noyées dans une couche de matrice extracellulaire, ladite unité étant obtenue à partir d'un microcompartiment cellulaire comprenant une capsule en hydrogel entourant ladite unité de tissu neural, et ladite capsule en hydrogel étant au moins partiellement éliminée avant implantation de l'unité de tissu neural.

De même, l'invention a pour objet une unité de tissu neural pour son utilisation pour le traitement de la maladie de Huntington par implantation dans le système nerveux d'un mammifère humain ou non humain, présentant la maladie de Huntington, d'au moins une unité de tissu neural formant un amas de cellules neurales, contenant des cellules neuronales post-mitotiques différenciées en neurones GABAergiques immuno-compatibles avec le mammifère destiné à recevoir l'unité de tissu neural, organisées en trois dimensions et noyées dans une couche de matrice extracellulaire, ladite unité étant obtenue à partir d'un microcompartiment cellulaire comprenant une capsule en hydrogel entourant ladite unité de tissu neural, et ladite capsule en hydrogel étant au moins partiellement éliminée avant implantation de l'unité de tissu neural.

L'invention a également pour objet une méthode de traitement de la maladie de Huntington, selon laquelle on implante dans le système nerveux d'un patient présentant la maladie de Huntington au moins une unité de tissu neural formant un amas de cellules neurales, contenant des cellules neuronales post-mitotiques différenciées en neurones GABAergiques immuno-compatibles avec le mammifère destiné à recevoir l'unité de tissu neural, organisées en trois dimensions et noyées dans une couche de matrice extracellulaire, ladite unité étant obtenue à partir d'un microcompartiment cellulaire comprenant une capsule en hydrogel entourant ladite unité de tissu neural, et ladite capsule en hydrogel étant au moins partiellement éliminée avant implantation de l'unité de tissu neural.

De manière similaire, l'invention a pour objet une unité de tissu neural formant un amas de cellules neurales pour son utilisation pour une implantation dans le système nerveux d'un mammifère humain ou non humain, dans laquelle ladite unité de tissu neural, capable de projeter des axones au sein du tissu hôte, contient des neurones modifiés génétiquement immuno-compatibles avec le mammifère destiné à recevoir l'unité de tissu neural, organisées en trois dimensions et noyées. De telles unités de tissu neural peuvent notamment être utilisées pour assister, améliorer ou réparer une fonction de cognition et/ou d'action chez un mammifère humain ou non humain.

L'unité de tissu neural selon l'invention peut être obtenu par un procédé de préparation d'une telle unité de tissu neural, destinée à être implantée dans le système nerveux d'un mammifère humain ou non humain, ledit procédé comprenant les étapes consistant à :
(1) produire des microcompartiments cellulaires comprenant, à l'intérieur d'une capsule d'hydrogel, de la matrice extracellulaire et des cellules de mammifère humain ou non humain, aptes à se différencier en cellules neurales, avantageusement immuno-compatibles avec le mammifère destiné à recevoir l'unité de tissu neural ;
(2) induire la différenciation cellulaire au sein du microcompartiment cellulaire, de manière à obtenir des cellules neuronales présentant au moins un phénotype post-mitotique d'intérêt ;
(3) éliminer au moins partiellement la capsule d'hydrogel pour récupérer les cellules neuronales sous forme d'unité de tissu neural.

L'invention concerne également un kit d'implantation d'unités de tissu neural comprenant au moins un microcompartiment cellulaire comprenant au moins une capsule d'hydrogel enveloppant une unité de tissu neural selon l'invention, et des moyens d'élimination au moins partielle de la capsule d'hydrogel.

L'invention concerne également un kit d'implantation d'unités de tissu neural comprenant au moins une unité de tissu neural selon l'invention, préférentiellement entre 10 et 100 et jusqu'à 1000, lesdites unités de tissu neural étant chargées dans un dispositif d'implantation chirurgical apte à implanter les unités de tissu neural dans le système nerveux d'un mammifère.

### Description des figures

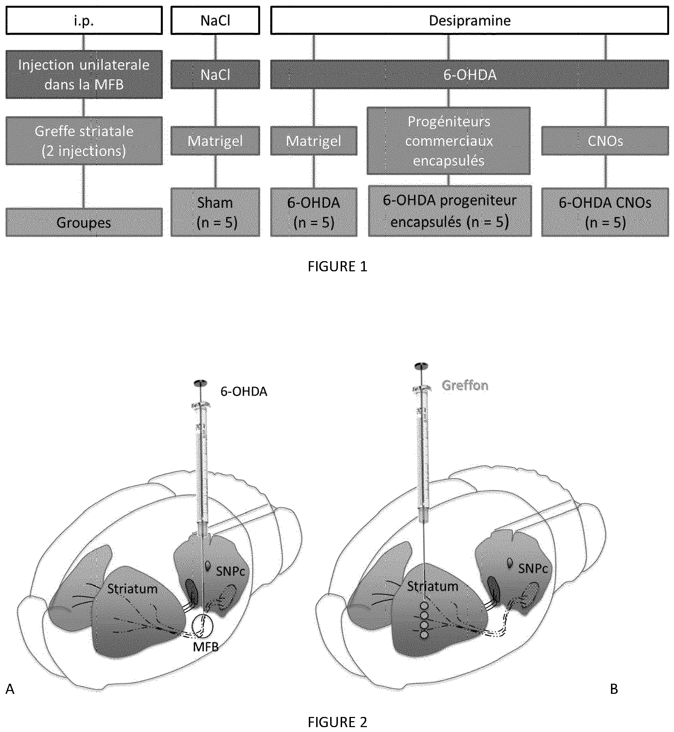

La figure 1 est une représentation schématique du protocole de préparation de différents rats rendus parkinsoniens, traités ou non par injection de tissu neural selon l'invention. Sham : rats contrôles ; 6-OHDA : rats rendus parkinsoniens (témoin négatif) ; 6-OHDA progeniteur encapsulés (ou Pro encaps) : rats rendus parkinsoniens ayant reçu des unités de tissu neural selon l'invention, provenant de microcompartiments cellulaires dans lesquels des cellules progénitrices de neurones commerciales ont été encapsulées ; 6-OHDA CNOs (Controlled Neural Organoids) : rats rendus parkinsoniens ayant reçu des unités de tissu neural (qui correspondent dans cet exemple aux CNOs) selon l'invention, provenant de microcompartiments cellulaires dans lesquels des cellules pluripotentes ont été encapsulées avant d'être différenciées en neurones, parmi lesquels des neurones dopaminergiques ;
Les figures 2A et 2B décrivent le protocole de lésion (2A) et de greffe (2B) appliqué pour obtenir des rats parkinsoniens et pour greffer les tissus neuronaux selon l'invention ;

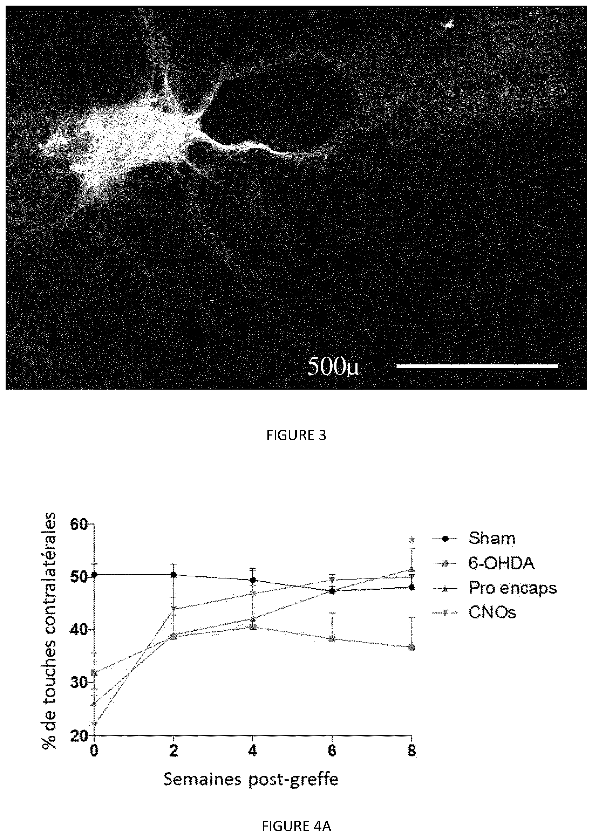

La figure 3 est une micrographie montrant les projections axonales pluri-millimétriques de neurones humains 6 jours après la greffe d'une unité neurale implantable selon l'invention (dans cet exemple de type CNO) dans le système nerveux central d'un rat rendu parkinsonien (6-OHDA CNOs). Il s'agit d'une imagerie confocale d'une section de striatum de rat dans la zone de greffe, fixée, congelée et coupée au cryostat, révélée par histochimie avec un anticorps contre le cytoplasme humain (HCM) ;
Les figures 4A-E montrent les résultats obtenus pour les tests comportementaux réalisés sur des rats rendus parkinsoniens ayant ou non reçu une greffe d'unité de tissu neural selon l'invention. (4A) test du cylindre ; (4B) Test de stepping ; (4C) test du rotomètre ; (4D) Test d'anxiété.

### Description détaillée

La présente invention divulgue une méthode d'implantation, ou greffe, dans le système nerveux d'un mammifère, d'une unité de tissu neural dans laquelle des cellules neurales sont organisées en réseau 3D. La greffe des cellules neurales sous forme de boules denses favorise leur implantation et leur intégration au sein du système nerveux hôte. La méthode d'implantation selon l'invention, et plus généralement l'unité de tissu neural mise au point selon l'invention, est destinée à être utilisée chez un mammifère humain ou non humain, et plus préférentiellement chez un sujet humain.

### Unité de tissu neural

L'invention propose d'utiliser, en lieu et place d'une suspension de cellules neuronales, une unité de tissu neural, pour son implantation dans le système nerveux d'un sujet, afin de traiter toutes sortes de maladies neurodégénératives et/ou d'assister, améliorer, réparer une fonction de cognition ou d'action chez ledit sujet.

Selon l'invention, l'unité de tissu neural destinée à être implantée comprend avantageusement des neurones post-mitotiques différenciés et des cellules gliales, telles que des astrocytes et/ou des oligodendrocytes dans une matrice extracellulaire.

Par neurones « post-mitotiques », on entend des cellules neuronales qui ont perdu la capacité de se diviser. Les neurones contenus dans le microcompartiment sont différenciés en ce sens qu'ils présentent un phénotype particulier.

Selon l'invention, la capsule d'hydrogel entourant l'unité de tissu neural est au moins partiellement éliminée avant l'implantation dans le système nerveux du sujet.

Dans le contexte de l'invention, la « capsule d'hydrogel » désigne une structure tridimensionnelle formée à partir d'une matrice de chaînes de polymères gonflée par un liquide, et préférentiellement de l'eau. Avantageusement, l'hydrogel utilisé est biocompatible, en ce sens qu'il n'est pas toxique pour les cellules. En outre, la capsule d'hydrogel doit permettre la diffusion d'oxygène et de nutriments pour alimenter les cellules contenues dans le microcompartiment et permettre leur survie. Par exemple, la capsule d'hydrogel contient de l'alginate. Préférentiellement, la capsule ne comprend que de l'alginate. Dans le contexte de l'invention, on entend par « alginate » des polysaccharides linéaires formés à partir de β-D-mannuronate et α-L-guluronate, des sels et dérivés de ceux-ci. Avantageusement, l'alginate est un alginate de sodium, composé à 80% de α-L-guluronate et 20% de β-D-mannuronate, avec une masse moléculaire moyenne de 100 à 400 KDa (par exemple : PRONOVA^{™} SLG100) et une à concentration totale comprise entre 0.5% et 5% en masse.

La capsule d'hydrogel permet au moment de la préparation de l'unité de tissu neural de protéger les cellules du milieu extérieur tout en permettant leur différenciation contrôlée en un ou plusieurs types neuronaux d'intérêt. Selon l'invention, une capsule d'hydrogel entoure une unique unité de tissu neural. Ainsi, chaque unité de tissu neural est entourée individuellement par une capsule d'hydrogel. Une fois le ou les types cellulaires voulus obtenus et la taille du tissu neural dans la capsule suffisante, ladite capsule est au moins partiellement éliminée. Par « au moins partiellement éliminée », on entend que la capsule d'hydrogel est au moins partiellement hydrolysée, dissoute, percée, ou rompue de manière à permettre au moins aux axones d'au moins certaines cellules neurales de ladite unité de sortir de la capsule. Tout moyen biocompatible, c'est-à-dire non toxique pour les cellules, permettant l'hydrolyse, la dissolution, le perçage et/ou la rupture au moins partielle de la capsule d'hydrogel peut être utilisé. Par exemple, il est possible d'utiliser du tampon phosphate salin, un chélateur d'ions divalents, une enzyme, telle que l'alginate lyase, la microdissection laser, des billes solubles intégrées dans l'hydrogel, etc.

Avantageusement, l'élimination de la capsule est totale, l'unité de tissu neural destinée à être implantée dans le système nerveux d'un sujet étant ainsi dépourvue d'hydrogel.

Préférentiellement, la couche de matrice extracellulaire de l'unité de tissu neural forme un gel. La couche de matrice extracellulaire comprend un mélange de protéines et de composés extracellulaires nécessaires à la culture cellulaire, et plus particulièrement à la culture de cellules neurales. Préférentiellement, la matrice extracellulaire comprend des protéines structurelles, telles que des laminines contenant les sous-unités α1 ou α4 ou α5 et β1 ou β2 et γ1 ou γ3, de l'entactine, de la vitronectine, du collagène, ainsi que des facteurs de croissance, tels que du TGF-béta et/ou de l'EGF. Dans un mode de réalisation, la couche de matrice extracellulaire consiste en, ou contient du Matrigel^{®} et/ou de la Geltrex^{®}.

L'unité de tissu neural selon l'invention présente l'avantage de contenir des cellules neurales organisées en réseau 3D au sein duquel des interactions entre cellules et matrice extracellulaire existent déjà.

Avantageusement, les cellules neurales de l'unité de tissu neural forment un amas de cellules neurales, notamment de forme ovoïde, tubulaire ou sphérique, présentant de manière préférée une plus petite dimension comprise entre 10 µm et 1000 µm plus ou moins 10%, préférentiellement entre 150 µm et 400 µm plus ou moins 10%, encore plus préférentiellement entre 200 µm et 300 µm plus ou moins 10%. Ces dimensions sont particulièrement favorables à la survie des neurones au sein de l'unité de tissu neural et optimisent la réorganisation ainsi que la vascularisation du greffon après implantation. Par « plus petite dimension », on entend la distance minimale entre deux points situés de part et d'autre de l'amas de cellules.

Préférentiellement, l'unité de tissu neural selon l'invention contient de 100 à 100.000 cellules neurales.

Au sein du tissu neural, les cellules neuronales présentent avantageusement un ou des phénotypes d'intérêt, choisis en fonction de l'utilisation à laquelle ladite unité de tissu neural est destinée. Selon l'invention, l'unité de tissu neural implantée comprend des cellules neuronales différenciées selon un ou des phénotypes choisis et contrôlés. Préférentiellement, l'unité de tissu neural comprend entre 10 et 100% de cellules neuronales post-mitotiques de phénotype(s) d'intérêt, préférentiellement entre 50 et 100%, plus préférentiellement plus de 90%. Les autres cellules présentes dans l'unité de tissu neural peuvent notamment être des cellules gliales et/ou des cellules neuronales post-mitotiques dont le phénotype est différent du phénotype d'intérêt. Avantageusement, l'unité de tissu neural comprend essentiellement des cellules neuronales post-mitotiques ayant le même phénotype.

Le phénotype d'intérêt des cellules neuronales post-mitotiques est fonction de la destination de ladite unité. Ainsi, dans le cas d'une utilisation en thérapie cellulaire, le phénotype des neurones correspond avantageusement au phénotype des cellules défaillantes à remplacer.

En effet, l'unité de tissu neural selon l'invention peut avantageusement être utilisée en thérapie cellulaire, pour le traitement d'une maladie neurodégénérative, telle que la maladie de Parkinson, la maladie de Huntington, la sclérose latérale amyotrophique (ALS), la maladie d'Alzheimer ou les céroïdes-lipofuscinoses neuronales, telles que la maladie de Batten.

Ainsi, pour le traitement de la maladie de Parkinson, l'unité de tissu neural contient avantageusement des neurones dopaminergiques. Préférentiellement, les neurones post-mitotiques contenus dans une telle unité de tissu neural sont essentiellement des neurones dopaminergiques.

Par « essentiellement », on entend dans le contexte de l'invention au moins 90% des cellules, préférentiellement au moins 95%, encore plus préférentiellement au moins 99%.

Pour le traitement de la maladie de Huntington, l'unité de tissu neural contient avantageusement des neurones GABAergiques. Préférentiellement, les neurones post-mitotiques contenus dans une telle unité de tissu neural sont essentiellement des neurones GABAergiques.

L'unité de tissu neural selon l'invention peut également contenir des cellules neurales modifiées génétiquement. Ainsi, il est possible d'implanter au sein du système nerveux d'un sujet des cellules neurales dans lesquelles l'expression d'un ou plusieurs gènes a été inhibée ou au contraire accrue. Il est également possible d'implanter des cellules neurales dans lesquelles un gène hétérologue a été introduit, de manière à introduire au sein du système nerveux une fonction normalement absente.

Une telle unité de tissu neural, contenant des cellules modifiées génétiquement, trouve un intérêt notamment pour assister, améliorer ou réparer une fonction de cognition et/ou d'action, telle qu'une fonction de cognition et/ou d'action défaillante ou insuffisante chez le sujet considéré.

Par exemple, dans le traitement de la maladie de Huntington, la cause génétique étant connue (élongation d'une répétition de triplet CAG au sein du gène codant pour la huntigtine), il est possible de prélever des cellules du patient pour préparer des unités de tissu neural selon l'invention, comprenant des neurones GABAergiques dans lesquelles le gène déficient a été corrigé, avant de pratiquer une autogreffe desdites unités de tissu neural.

Pour les patients atteints de la maladie de Parkinson, il est possible de préparer des unités de tissu neural comprenant des neurones dopaminergiques modifiés pour exprimer des canaux sensibles à la lumière, tels que les canaux rhodopsine, afin de contrôler l'activité du tissu neural greffé par simple illumination, de manière similaire à la stimulation cérébrale profonde (deep brain stimulation) mais moins invasive. D'une manière générale, le contrôle de l'activité du tissu neural greffé, notamment par expression de canaux sensibles à la lumière, peut être avantageux pour l'ensemble des applications envisagées.

### Kits d'implantation d'unités de tissu neural

L'invention propose également un kit d'implantation d'unités de tissu neural comprenant au moins un microcompartiment cellulaire comprenant une capsule d'hydrogel enveloppant une unité de tissu neural selon l'invention, et des moyens d'élimination au moins partielle de la capsule d'hydrogel (hydrolyse, dissolution, perçage et/ou rupture).

Le praticien devant utiliser l'unité de tissu neural selon l'invention peut ainsi, au moment de l'utilisation et en fonction des besoins, éliminer au moins partiellement la capsule d'hydrogel pour obtenir la ou les unités de tissu neural prêtes à être implantées dans le système nerveux d'un sujet.

Aussi, le kit d'implantation selon l'invention peut également contenir un dispositif d'implantation chirurgical apte à implanter une unité de tissu neural dans le système nerveux d'un mammifère.

L'invention propose également un kit d'implantation d'unités de tissu neural comprenant au moins une unité de tissu neural selon l'invention, préférentiellement entre 1 et 10.000, plus préférentiellement entre 10 et 1000 unités de tissu neural chargée(s) dans un dispositif d'implantation chirurgical apte à implanter la ou les unités de tissu neural dans le système nerveux d'un mammifère. Avantageusement, la quantité d'unités de tissu neural implantée est fonction de la taille du cerveau hôte et de l'application.

Les unités de tissu neural peuvent éventuellement être congelées, avant d'être introduites dans le dispositif d'implantation chirurgical et/ou être congelées en même temps que le dispositif d'implantation chirurgical. Bien entendu, une étape de décongélation est alors nécessaire avant l'implantation des unités de tissu neural dans le système nerveux du sujet.

### Procédé de préparation

L'unité de tissu neural aptes à être implantées dans le système nerveux d'un mammifère selon l'invention peut être préparé par un procédé de préparation d'unités de tissu neural. Plus particulièrement, l'invention propose de réaliser des unités de tissu neural contenant des cellules neuronales post-mitotiques dont le ou les phénotypes sont fonction de l'utilisation à laquelle lesdites unités sont destinées. Selon l'invention, il est possible de réaliser des microcompartiments cellulaires comprenant une capsule d'hydrogel entourant des cellules neurales, notamment à partir de cellules différenciées de mammifères auxquelles on fait subir une reprogrammation avant ou après encapsulation, puis qui sont différenciées en un ou plusieurs types neuraux au sein de la capsule d'hydrogel. La capsule est ensuite au moins partiellement éliminée, afin de permettre aux cellules neurales de s'implanter dans le tissu hôte après la greffe.

D'une manière générale, le procédé de préparation d'une telle unité de tissu neural, destinée à être implantée dans le système nerveux d'un mammifère humain ou non humain, selon l'invention comprend les étapes consistant à :
(1) produire des microcompartiments cellulaires comprenant, à l'intérieur d'une capsule d'hydrogel, de la matrice extracellulaire et des cellules de mammifère humain ou non humain, aptes à se différencier en cellules neurales, avantageusement immuno-compatibles avec le mammifère destiné à recevoir l'unité de tissu neural ;
(2) induire la différenciation cellulaire au sein du microcompartiment cellulaire, de manière à obtenir des cellules neuronales post-mitotiques présentant au moins un phénotype d'intérêt ;
(3) éliminer au moins partiellement la capsule d'hydrogel, pour récupérer les cellules neurales sous forme d'unité de tissu neural.

Toute méthode de production de microcompartiments cellulaires contenant à l'intérieur d'une capsule d'hydrogel de la matrice extracellulaire et des cellules susceptibles de donner naissance à des cellules neurales peut être utilisée. Notamment, il est possible de préparer des microcompartiments avec le dispositif microfluidique décrit dans Alessandri et al., 2016 (« A 3D printed microfluidic device for production of functionalized hydrogel microcapsules for culture and differentiation of human Neuronal Stem Cells (hNSC) », Lab on a Chip, 2016, vol. 16, no. 9, p. 1593-1604).

Dans un mode de réalisation particulier, les cellules encapsulées sont des cellules souches pluripotentes, telles que des cellules souches pluripotentes induites (IPS), ou des cellules souches embryonnaires (ES). Dans le contexte de l'invention, les « cellules souches pluripotentes induites » (CSPI ou IPS), s'entendent des cellules souches pluripotentes obtenues par reprogrammation génétique de cellules somatiques différenciées, et présentant une morphologie et un potentiel d'autorenouvellement et de pluripotence en partie similaires à ceux des cellules souches embryonnaires. Ces cellules sont notamment positives pour les marqueurs de pluripotence, comme la coloration à la phosphatase alcaline et l'expression des protéines NANOG, SOX2, OCT4 et SSEA3/4. Les procédés permettant l'obtention des cellules souches pluripotentes induites sont bien connus de l'homme du métier et sont notamment décrits dans les articles de Yu et al (Science, 2007, 318 (5858): 1917-1920), Takahashi et al (Cell, 2007, 131(5): 861-872) et Nakagawa et al (Nat Biotechnol, 2008, 26(1): 101-106). Dans le cas de cellules souches embryonnaires, lesdites cellules souches pluripotentes s'entendent des cellules dérivées de la masse cellulaire interne du blastocyste et qui ont la capacité de conduire à la formation de tous les tissus de l'organisme. La pluripotence des cellules souches embryonnaires peut être évaluée par la présence de marqueurs tels que les facteurs de transcription OCT4 et NANOG et des marqueurs de surface comme SSEA3/4, Tra-1-60 et Tra-1-81. Les cellules souches embryonnaires peuvent être obtenues sans destruction de l'embryon dont elles sont issues, par exemple à l'aide de la technique décrite par Chung et al. (Cell Stem Cell, 2008, 2(2): 113-117). Dans un mode de réalisation particulier, et pour des raisons légales ou éthiques, les cellules souches s'entendent à l'exclusion des cellules souches embryonnaires humaines.

De manière alternative, il est possible d'encapsuler des cellules progénitrices neurales, c'est-à-dire des cellules souches déjà engagées dans la différenciation cellulaire en cellules neurales. Dans un autre mode de réalisation, il est possible d'encapsuler des cellules différenciées, qui seront forcées à se différencier en cellules neurales par trans-différenciation. Il est également possible d'encapsuler des cellules aptes à former des cellules gliales, telles que des cellules somatiques.

Dans un autre mode de réalisation, il est possible d'utiliser des cellules différenciées, qui seront reprogrammées en cellules souches pluripotentes avant ou après encapsulation. Dans la mesure où les unités de tissu neural doivent pouvoir être implantées pendant un temps long dans le système nerveux d'un patient, il est préférable de partir de cellules immuno-compatibles avec ledit sujet, pour éviter tout risque de rejet. Dans un mode de mise en oeuvre particulier, les cellules utilisées pour préparer les unités de tissu neural ont été préalablement prélevées chez le mammifère humain ou non humain dans lequel la ou lesdites unités de tissu neural doivent être implantées.

Dans tous les cas, une fois les microcompartiments cellulaire obtenus, les cellules qu'il contient doivent subir une différenciation cellulaire de manière à les différencier notamment en cellules neuronales selon un ou plusieurs phénotypes souhaités.

Toute méthode mise en oeuvre classiquement en culture 2D (boîte de pétri et autre) pour forcer la différenciation cellulaire peut être utilisée, telle que la méthode décrite dans Chambers et al. ("Highly efficient neural conversion of human ES and iPS cells by dual inhibition of SMAD signaling", Nature Biotechnology 27, 275 - 280 (2009)), ou dans Lippmann et al., (« Defined human pluripotent stem cell culture enables highly efficient neuroepithelium dérivation without small molécule inhibitors », Stem Cells 2014). Dans un mode de mise en oeuvre particulier, le milieu de différenciation contient du 24(S),25-Epoxycholestérol, de manière à améliorer la différenciation dopaminergique.

Préférentiellement, les microcompartiments cellulaires sont cultivés pendant au moins trois semaines dans un milieu de différenciation et avant toute élimination de la capsule d'hydrogel.

D'une manière générale, les microcompartiments cellulaires pluripotents peuvent être congelés avant toute utilisation, et être décongelés selon les besoins.

Dans un exemple de réalisation, il est possible d'induire une différenciation cellulaire de manière à forcer les cellules, à l'intérieur du microcompartiment cellulaire, à se différencier en cellules dopaminergiques (« Generating regionalized neuronal cells from pluripotency, a step-by-step protocol » Kirkeby et al. Frontiers in Cellular Neuroscience 2012). Les unités de tissu neural issues de tels microcompartiments cellulaires peuvent ensuite être implantées dans le système nerveux d'un sujet atteint de la maladie de Parkinson, afin de remplacer au moins partiellement les neurones dopaminergiques défaillants dudit sujet.

Dans un autre exemple de réalisation, il est possible d'induire une différenciation cellulaire de manière à forcer les cellules, à l'intérieur du microcompartiment cellulaire, à se différencier en cellules GABAergiques (« Dérivation of striatal neurons from human stem cells » Viegas et al. Prog Brain Res 2012). Les unités de tissu neural issues de tels microcompartiments cellulaires peuvent ensuite être implantées dans le système nerveux d'un sujet atteint de la chorée de Huntington, afin de remplacer au moins partiellement les neurones GABAergiques défaillants dudit sujet.

Le procédé de préparation d'unités de tissu neural selon l'invention peut également comprendre l'étape supplémentaire consistant à :
- charger un dispositif d'implantation chirurgical avec au moins une unité de tissu neural, préférentiellement entre 10 à 1000, plus préférentiellement entre 10 et 100 unités de tissu neural.

Ainsi, le dispositif d'implantation est prêt à être utilisé pour greffer des cellules neurales dans le système nerveux d'un sujet.

Selon l'invention, il est possible de congeler les cellules neurales sous forme d'unités de tissu neural, avant ou après élimination au moins partielle de la capsule d'hydrogel. Bien entendu, les unités de tissu neural peuvent être utilisées directement à l'issue du procédé de préparation, sans avoir été congelées au préalable.

Avantageusement, le procédé de préparation selon l'invention comprend l'étape intermédiaire consistant à :
- vérifier le phénotype des cellules neurales contenues dans la capsule d'hydrogel, après l'étape de différenciation cellulaire.

Par exemple, dans le cas d'unité(s) de tissu neural comprenant des neurones dopaminergiques, il est possible de mesurer l'activité des unités neurales implantables avant la greffe de manière non invasive en prélevant le surnageant du milieu de culture, et en pratiquant une analyse par microdialyse couplée à une HPLC.

Plus généralement, il peut être avantageux de caractériser l'activité électrique des neurones représentatifs de l'échantillon par la technique dite de « Patch-Clamp » basée sur l'enregistrement des courants ioniques transitant à travers les membranes cellulaires.

Les unités de tissu neural étant avantageusement cultivées en lots, dans des conditions identiques, il est également possible de pratiquer des analyses plus poussées, notamment par immunocytochimie, séquençage des ARN, et/ou protéomique, sur un échantillon représentatif de la population des unités de tissu neural considéré, afin de s'assurer de la qualité exacte de la différenciation. Par exemple, dans le cas d'unités de tissu neural comprenant des neurones dopaminergiques, il est possible de procéder à une analyse par immunocytochimie ciblant la Tyrosine Hydroxylase, FOXA2 et/ou NURR1 (« Dopamine neurons derived from human ES cells efficiently engraft in animal models of Parkinson's disease » Kriks et al 2012).

Il est ainsi possible de vérifier que les neurones post-mitotiques et/ou les cellules neurales présentent bien le ou les phénotypes souhaités avant l'étape d'implantation dans le système nerveux du sujet.

### Greffe d'unité(s) de tissu neural dans le système nerveux d'un mammifère humain ou non humain

L'invention propose également un procédé d'implantation, ou de greffe, d'unité(s) de tissu neural dans le système nerveux d'un sujet, et notamment d'un humain, en ayant besoin. En effet, les unités de tissu neural selon l'invention peuvent avantageusement être utilisées pour le traitement de maladies neurodégénératives. Dans le cadre de l'invention, le terme « traitement » désigne un traitement curatif, symptomatique ou préventif. Tel qu'utilisé ici, le terme « traitement » d'une maladie se réfère à tout acte destiné à prolonger la qualité et/ou la durée de vie des patients. Le traitement peut être conçu pour éradiquer la maladie, arrêter ou retarder la progression de la maladie et / ou favoriser la régression de la maladie. Le terme « traitement » d'une maladie désigne également tout acte destiné à diminuer les symptômes associés à la maladie. Le patient à traiter est n'importe quel mammifère, de préférence un être humain.

D'une manière générale, toute méthode de greffe de cellules neurales peut être utilisée pour implanter les unités de tissu neural au sein du système nerveux du sujet. Notamment, il est possible d'utiliser une canule, telle qu'une canule en verre, dans laquelle ont été préalablement chargées les unités de tissu neural.

Dans un mode de mise en oeuvre particulier, l'implantation comprend les étapes consistant à
- Endormir et/ou immobiliser le sujet devant recevoir la ou les unités de tissu neural ;
- Ouvrir le crâne du sujet ;
- Positionner le dispositif d'implantation contenant les unités de tissu neural au-dessus de la zone de greffe, par exemple à l'aide d'un appareil dit « stéréotaxique »;
- Insérer le dispositif d'implantation dans la zone de greffe ;
- Injecter les unités de tissu neural tout en retirant progressivement le dispositif d'implantation de la zone de greffe.

Avantageusement, l'étape d'injection est contrôlée par un système de pompe micro-fluidique et de motorisation permettant de gérer la profondeur d'enfoncement du dispositif d'implantation dans le système nerveux du sujet, pour ménager la place nécessaire aux unités de tissu neural dans ledit système nerveux, qui sont ainsi déposées dans la trajectoire de retrait du dispositif d'implantation.

Dans un mode de mise en oeuvre particulier, le dispositif d'implantation consiste en une canule en verre présentant un diamètre interne compris entre 0,3 et 2 mm, préférentiellement 0,4 mm, et un diamètre externe compris entre 0,4 et 3 mm, préférentiellement 0,55 mm. La canule présente avantageusement un arrondi ou un chanfrein à son extrémité et sa surface est silanisée avec un composé fluoré, afin de réduire les risques de dommages aux tissus lors de l'insertion de la canule et réduire les risques de bouchons par un caillot sanguin. Les unités de tissu neural sont chargées, par exemple par l'avant, dans la canule. La canule est insérée en position dans le crâne du sujet.

Avantageusement, lors d'une même greffe, entre 10 et 40 unités de tissu neural sont implantées, chaque unité de tissu neural comprenant préférentiellement entre 1.000 et 10.000 cellules neurales. Bien entendu, le nombre d'unités de tissu neural à implanter est adapté en fonction du sujet et de la maladie à traiter et/ou de la fonction de cognition ou d'action à modifier. Plusieurs greffes, plus ou moins éloignées dans le temps peuvent également être envisagées. De même, des greffes simultanées ou dans un laps de temps court peuvent être réalisées dans différentes zones du système nerveux du sujet.

### EXEMPLES

### 1. Fabrication d'unités de tissu neural implantables, pour le traitement de la maladie de Parkinson :

Des progéniteurs sont obtenus d'après la méthode publiée par Kriks et al. (« Dopamine neurons derived from human ES cells efficiently engraft in animal models of Parkinson's disease » Kriks et al 2012), en utilisant du 24(S),25-Epoxycholesterol (concentration de 1 à 10µM) sur des cultures issues de cellules souches induites pluripotentes humaines, pour optimiser la différenciation en neurones dopaminergiques.

Les progéniteurs ainsi obtenus sont encapsulés d'après la méthode publiée par Alessandri et al., 2016 (« A 3D printed microfluidic device for production of functionalized hydrogel microcapsules for culture and differentiation of human Neuronal Stem Cells (hNSC) », Lab on a Chip, 2016).

Une fois les microcompartiments cellulaires contenant les progéniteurs obtenus, une différenciation dite « terminale » en neurones dopaminergiques est effectuée d'après la méthode publiée par Kriks et al. (« Dopamine neurons derived from human ES cells efficiently engraft in animal models of Parkinson's disease » Kriks et al 2012), en utilisant à nouveau du 24(S),25-Epoxycholesterol (concentration de 1 à 10µM).

Une attention particulière est portée au changement des milieux de culture, car la prolifération des cellules dans les microcompartiments cellulaires augmente la vitesse à laquelle le milieu est consommé par les unités neurales implantable au cours de leur préparation.

L'ensemble de la différenciation a lieu dans les microcompartiments cellulaires, mais il est possible de décapsuler et de réencapsuler les cellules, tant que les cellules supportent la dissociation en suspension de cellules uniques (soit jusqu'à maximum une semaine après ajout d'inhibiteur de gamma sécrétase et/ou du signal Notch tel que le DAPT, le CoumpoundE ou d'autres molécules poussant à la différenciation terminale).

Les microcompartiments cellulaires ainsi obtenus après trois semaines de différenciations sont traitées par rinçages successifs dans une solution de PBS pour dissoudre la coque en hydrogel et récupérer les unités de tissu neural avant injection.

### Fabrication de la canule d'injection :

Une section de capillaire en borosilicate (diamètre interne : 0,4mm, diamètre externe 0,55mm) de 5cm est coupée à l'aide d'une lame de céramique.

La face avant du capillaire est arrondie à l'aide d'un papier de ponçage d'oxyde d'aluminium pour fibre optique 12µm puis 1µm.

Le verre est activé via un traitement au plasma pendant 5 minutes, puis silanisé en phase gazeuse par dépôt de 20 µl de 1H,1H,2H,2H-Perfluorooctyltrichlorosilane près de la pointe positionnée dans une boite de Pétri en verre hermétiquement fermée.

Après 30 minutes de réaction, la canule d'injection est prête et montée sur un dispositif standard de stéréotaxie lié à une seringue 10µl (hamilton), motorisée grâce à un pousse seringue (harvard apparatus). Le bras d'injection de l'appareil stéréotaxique est également motorisé (PI).

### Chirurgie :

Une chirurgie standard pour la greffe de neurones est alors pratiquée sur un rat. Une telle méthode chirurgicale comprend les étapes selon lesquelles :
1) On induit un sommeil médicamenteux (kétamine) et une anesthésie chez l'animal
2) On positionne précisément l'animal grâce à un appareillage stéréotaxique.
3) On rase le crâne de l'animal, traité ensuite à la bétadine, puis ouvert à l'aide d'un scalpel pour écarter la peau du crâne, puis une micro-perceuse est utilisée pour former une fenestration dans l'os du crâne, permettant le passage de la canule. Les méninges sont précautionneusement écartées sans abîmer le cortex qui affleure juste en dessous.
4) On positionne la canule reliée à une seringue à l'aide d'un appareil stéréotaxique au-dessus de la zone de greffe.
5) On charge les unités de tissu neural par l'avant dans la canule (environ 80 capsules soit 10.000 à 800.000 cellules). La greffe représente 4µl au total soit 4cm de hauteur dans la canule. Deux sites sont injectés unilatéralement chez le rat, dans le striatum à environ 1mm de distance.
6) On insère la canule en position dans le crâne de l'animal dans le premier site au sein du striatum ventral. Durant l'injection de 2µl, la canule est retirée simultanément à l'injection grâce à un système de pompe micro-fluidique et de gestion motorisée de la profondeur de la canule, pour libérer l'espace nécessaire pour le greffon qui est ainsi déposé dans la trajectoire de retrait sur environ 2mm.
7) On laisse la canule en place pour une minute avant de la retirer doucement.
8) On procède ensuite à l'implantation dans le deuxième site, selon la même procédure.
9) On suture la peau du rat (trois à cinq points) après avoir rincé la plaie à la bétadine.

### Résultats de la greffe :

La démonstration étant la survie au processus de greffe des neurones matures humains, l'animal est sacrifié une semaine après la chirurgie pour étudier la survie et l'implantation de la greffe par immunocytochimie sur une tranche de cerveau découpée au vibratome dans la trajectoire de greffe.

La survie estimée d'après la taille des greffons (connue avant la greffe) est supérieure à 84% ± 33%, comparativement au taux de survie de 2% obtenu avec les méthodes de greffage de neurones matures dopaminergiques en suspension (D. M. Marchionini et al., « Interférence with anoikis-induced cell death of dopamine neurons: implications for augmenting embryonic graft survival in a rat model of Parkinson's disease. » J Comp Neurol 464, 172-179 (2003).). Le phénotype des neurones est conservé (Tyrosine Hydroxylase positif). En outre, des processus axonaux de plusieurs millimètres sont visibles, indiquant que les neurones ont immédiatement commencé à s'intégrer dans le cerveau du rat.

### 2. Greffe d'unités de tissu neural comprenant des cellules neuronales pour le traitement de Parkinson

### 2.1- Protocole chirurgical

Des rats (âgés de 7/8 semaines) sont hébergés dans une pièce à température contrôlée dans un cycle de lumière / obscurité de 12 h avec un accès *ad libitum* à la nourriture et à l'eau.

La figure 1 résume les différentes interventions pratiquées sur ces rats afin d'obtenir des rats parkinsoniens, auxquels vont être appliquées ensuite des greffes de tissu neural selon l'invention.

### Préparation des rats parkinsoniens

Les animaux sont mis à jeuner avant la chirurgie. Les aliments, y compris les suppléments alimentaires, et l'eau sont retenus.

Chaque animal est placé dans une chambre d'anesthésie alimentée par un flux continu d'air (0,3L / min) + oxygène (0,3L / min) et 2% d'isoflurane. De la xylocaïne (7 mg / kg, s.c.), du borgal (7,5%, i.m.) et de la buprénorphine (0,1 mg / kg, s.c.) sont injectés. L'animal est ensuite maintenu sous anesthésie avec un anesthésique à base d'isoflurane administré par un respirateur à régulation de volume. La température centrale est enregistrée par voie rectale pendant la chirurgie. Des coussinets chauffants sont utilisés pour maintenir la température du corps, si nécessaire. Une pommade ophtalmique (gel ophtalmique liposique) est administrée à chaque oeil. Les poils sont coupés du crâne au cou. Après la perte de conscience, l'animal est placé dans un cadre stéréotaxique (Kopf) et sa tête fixée en position à l'aide de barres d'oreille.

A l'aide d'une seringue Hamilton 1702N (Ga22s / 51mm / PST3) couplée à un injecteur automatique Legato 101 (KD scientific), les rats sont rendus pseudo-parkinsoniens par une injection stéréotaxiques unilatérale de 2,5µl de 6-hydroxydopamine (6-OHDA) (Sigma, 5mg / ml en NaCl stérile, 0, 9%) avec 0,1% d'acide ascorbique dans la MFB (Medial Forebrain Bundle) aux coordonnées -2,8 mm antéropostérieur, 2 mm latéral et 8,4 mm dorsoventral selon l'atlas cérébral de Paxinos et Watson à un débit de 1µl / min (figure 2A).

Après l'injection, l'aiguille est laissée en place pendant 5 minutes avant d'être retirée lentement du cerveau. Trente minutes avant la chirurgie, les animaux reçoivent en i.p. (intra-péritonéal) une injection de désipramine (25 mg / kg, 5 ml / kg) dissous dans 0,9% de NaCl, pour protéger le système noradrénergique.

Lorsque l'injection est terminée, la peau est fermée avec une suture. L'animal est ensuite autorisé à récupérer de l'anesthésie.

### Greffes

La préservation du microenvironnement des neurones matures tout au long de la procédure de greffe permet la survie et la connexion avec le cerveau hôte.

Un groupe de rats (6-OHDA progéniteurs encapsulés) reçoit une greffe d'unités de tissu neural selon l'invention comprenant des cellules progénitrices de neurones commerciales (Cellular dynamics icell dopaneurons) encapsulées et maturées pendant deux semaines in vitro avant utilisation.

Un second groupe de rats (6-OHDA CNOs) reçoit une greffe d'unités de tissu neural selon l'invention comprenant des CNO (Controlled Neural Organoid) produits et triés dans le laboratoire.

Les unités de tissu neural comprenant des cellules CNOs ont été obtenues en encapsulant dans de l'alginate des cellules pluripotentes ou des cellules pluripotentes naïves et en les différenciant en cellules progénitrices de neurones selon le protocole ci-dessous :
Toute la culture est réalisée à 37°C, sous 5% de CO2, et saturation d'humidité supérieure à 95%
J0-J3 (jour 0 à jour 3) : N2B27 (pour 500mL de milieu : 250mL de milieu Neurobasal + 250mL de milieu Dmem F12 avec glutamax+ 1complément N2 + 1 complément B27 + 0,5microM LDN-193189 + 10 microM SB431542 + 100ng/mL Shh (Sonic Hedgehog)+ 100ng/mL FGF-8 (Fibroblast Growth Factor 8) + 2microM purmorphamine+ 10 microM 24(S),25-Epoxycholesterol
J3-J9: N2B27 + LDN + SB + Shh + FGF-8 + purmorphamine + CHIR + 24(S),25-Epoxycholesterol
J9-J10: N2B27 + LDN + CHIR + 24(S),25-Epoxycholesterol
J10-J15 ou plus: N2B27 + AMPc + AA + GDNF + BDNF + FGF-20 + TGFbeta + trichostatine + CpE + 24(S),25-Epoxycholesterol

**Tableau 1: liste des abréviations et acronymes**

| **Abréviation / acronyme** | **Description** |
|---|---|
| AA | Acide Ascorbique |
| AMPc | Cyclic adenosine monophosphate - intracellular signal transduction |
| BDNF | Brain Derived Neurotrophic Factor |
| CHIR | GSK3 inhibitor |
| DMEM | Dulbecco's Modified Eagle Medium |
| DMSO | Dimethyl Sulfoxide |
| ECM | Extra cellular matrix |
| FGF-20 | Fibroblast growth factor 20 |
| FGF-8 | Fibroblast growth factor 8 |
| GDNF | Glial Derived Neurotrophic Factor |
| hESC | Human Embryonic Stem Cells |
| HS415 | Human ES cell line |
| LDN193189 | smad1/8/5/8 inhibitor |
| NEAA | Non-Essential Amino Acids |
| PBS | Phosphate Buffered Saline |
| PS | Penicillin / Streptomycin |
| RT | Room Température |
| SB431542 | Anti TGF beta |
| Shh | Sonic hedgehog |
| TGFbeta3 | transforming growth factor type 3 |

**Tableau 2 : Concentrations finales**

| **Petites moélcules ou facteurs de croissance** | **Concentrations finale au niveau des cellules** |
|---|---|
| AMPc | 0,5 mM |
| Ascorbic acid AA | 200 µM |
| BDNF | 10 ng/ml |
| Chir99021 | 3 µM |
| CompoundE | 1 µM |
| FGF-20 | 5 ng/ml |
| FGF-8 | 100 ng/ml |
| GDNF | 10 ng/ml |
| LDN -193189 | 0,5 µM |
| purmorphamine | 2 µM |
| rock inhibitor | 10 µM |
| SB431542 | 10 µM |
| Shh | 100 ng/ml |
| TGFbeta3 | 1 ng/ml |
| Tricostatin A | 10 nM |
| 24(S),25-Epoxycholesterol | 10 µm |

Avant l'injection des unités de tissu neural (6-OHDA progéniteurs encapsulés et 6-OHDA CNOs), les capsules d'alginate des microcompartiments cellulaires sont dissoutes simplement en échangeant le milieu avec du PBS.

Les configurations d'injection sont une seringue ou une canule montée sur un équipement stéréotaxique. Le chargement des unités de tissu neural dans la canule d'injection se fait par aspiration du volume injecté (25 CNO ~ 1µL) dans le corps de la canule.

Pour injecter les 4 sites d'injection sur 2 trajectoires (figure 2B) : 4 µl avec progéniteurs (groupe 3), progéniteurs encapsulés (6-OHDA progeniteur) ou CNOs (6-OHDA CNOs) sont injectés dans le striatum aux coordonnées suivantes : A / P +1.2; M / L -2,6; D / V-5,0 (3 ul) et -4,0 (3 ul); et A / P +0,5; M / L -3,0; D / V-5,0 (3 ul) et -4,0 (3 ul); barre de dent -2,4 (Grealish et al., 2014).

### Procédure post-opératoire

Quatre groupes de rats sont formés à partir des rats immunodéprimés (RNU ou rats nus) :
- « Sham » : rats non lésés
- « 6-OHDA » : rats dont les neurones dopaminergiques de l'hémisphère droit ont été chimiquement tués, pas de greffe (expérience contrôle)
- « CNO » ou « 6-OHDA CNO » : rats dont les neurones dopaminergiques de l'hémisphère droit ont été chimiquement tués, et greffés dans le striatum droit avec des unités de tissu neural selon l'invention comprenant environ 250 000 cellules.
- « Pro encaps » ou « 6-OHDA progéniteurs encapsulés » : rats dont les neurones dopaminergiques de l'hémisphère droit ont été chimiquement tués, et greffés dans le striatum droit avec unités de tissu neural selon l'invention comprenant environ 250 000 cellules de iDopaneurons de Cellular dynamics maturés 2 semaines en capsules.

Chaque animal est observé de près et maintenu au chaud jusqu'à ce qu'il ait retrouvé les réflexes de redressement et de déglutition. Les animaux sont pris en charge par des techniciens expérimentés ayant facilement accès à un soutien vétérinaire expérimenté.

D'autres médicaments pour la gestion clinique de l'animal sont utilisés si nécessaire. Chaque médicament, dose, voie et site d'administration est documenté dans les dossiers chirurgicaux.

Les incisions chirurgicales sont surveillées pour détecter des signes d'infection, d'inflammation et d'intégrité générale au moins une fois par jour (jusqu'à ce que les incisions soient cicatrisées). Toutes les observations effectuées et les résultats sont documentés dans les dossiers chirurgicaux. Le coordonnateur de l'étude est informé de toute anomalie. Le moniteur de l'étude est informé dès que raisonnablement possible de toute anomalie.

Des contrôles sur l'état des animaux sont effectués deux fois par jour (de 11h00 à 22h00).

Les animaux sont évalués selon les critères de traitement juste et humain. Un soutien vétérinaire approprié est fourni si nécessaire. Tout animal présentant des signes de douleur ou de détresse sévères, susceptibles de durer, est euthanasié rapidement. Ils sont ensuite soumis à une nécropsie grossière. Outre la nécropsie, le cerveau est prélevé pour l'histologie et traité comme indiqué dans la section Euthanasie. Tous les efforts raisonnables sont déployés pour appliquer les critères de traitement juste et humain afin d'éviter qu'un animal soit retrouvé mort.

### 2.2- Etudes comportementales

### Rotation induite par l'amphétamine (protocole adapté de Grealish et al., 2014).

Seuls les animaux ayant plus de 5 tours par minute après injection de 6-hydroxydopamine (6-OHDA) au niveau du faisceau télencéphalique médian ont été considérés comme ayant subi une lésion avec succès. Le biais de rotation, après une injection systémique d'amphétamine (2,5 mg/kg, par voie intrapéritonéale, Apoteksbolaget), a été enregistré en utilisant un système automatisé (Omnitech Electronics). La rotation des animaux a été enregistrée pendant 90 minutes, seuls les tours complets du corps ont été comptés et sont ensuite exprimés en tours nets par minute, les rotations vers le côté de la lésion (sens horaire) sont comptabilisées positivement.

### Test de stepping

Chaque rat est placé sur une surface plane ; ses pattes arrières sont soulevées en tenant doucement la queue de façon permettre seulement à une patte avant de toucher la table. L'expérimentateur tire le rat en arrière d'un mètre à un rythme régulier. Les mouvements d'ajustement des pattes avant controlatérales et ipsilatérales sont comptabilisé indépendamment sur leurs trajets respectifs. Les données seront présentées en pourcentage de mouvement d'ajustement utilisant les pattes en calculant le ratio controlatérales / (controlatérales + ipsilatérales).

### Test du cylindre

Le test du cylindre est effectué en plaçant chaque animal dans un cylindre en verre et en comptant le nombre de touches sur la paroi du cylindre pendant 5 min des pattes ipsilatérales et controlatérales, le résultat est exprimé en calculant le ratio controlatérales / (controlatérales + ipsilatérales), sur un maximum de 20 touches.

### L'actimètre "champ ouvert"

L'activité locomotrice spontanée est mesurée à l'aide d'un actimètre photoélectrique (Actitrack, Panlab, S.L., Barcelone, Espagne). L'appareil consiste en une cage transparente reliée à une cellule photoélectrique. Les faisceaux lumineux détectent les mouvements, et le nombre total de détection de mouvement horizontal de chaque rat est enregistré chaque jour sur deux sessions successives de 10 minutes. Tous les tests dans l'actimètre sont effectués dans une pièce isolée entre 8h00 et 13h00. La première phase de trois jours permet une accoutumance. Le quatrième jour est considéré comme le jour du test. La première séance de 10 min est considérée comme l'habituation quotidienne. Seule l'activité locomotrice enregistrée pendant la seconde session de 10 minutes est utilisée pour l'analyse des données.

### 2.3- Evaluations d'étude post-mortem - analyse histologique

Après la fin des expériences, les rats sont sacrifiés par perfusion intracardiaque de paraformaldéhyde à 4%, les cerveaux prélevés, congelés dans de l'isopentane à - 45 ° C et conservés à - 80 ° C. Les cerveaux perfusés sont coupés au cryostat en sections coronales de 50 µm.

### Validation de la lésion dopaminergique (DA)

La perte des cellules DA dans le système nerveux central et la perte des fibres DA dans le striatum sont vérifiées par immunohistochimie de la tyrosine hydroxylase (TH) comme décrit précédemment (Bouali-Benazzouz et al., 2009). Le nombre de cellules immunoréactives à la Tyrosine Hydroxylase (TH) est obtenu en appliquant la méthode du fractionnement optique en utilisant un microscope Leica DM6000B avec le logiciel Mercator Pro (ExploraNova, version 7.9.8).

### Validation de la greffe

Les coupes de cerveaux de rat et les CNO sont soumis à des analyses immunohistochimiques utilisant des anticorps pour analyser la survie cellulaire, la prolifération possible et la maturation phénotypique des greffons (identifiés à l'aide de HCM -Human Cytoplasmic Marker-) et caractérisés par des marqueurs DA classiques (TH, DAT, FOXA2). , les marqueurs pan-neuronaux (MAP2, NeuN), sérotoninergiques (FOXG1), les marqueurs prolifératifs Ki-67 / PCNA et un marqueur glial (GFAP spécifique de l'homme).

### Test de survie neuronale

6 rats CNOs ont été sacrifiés 6 jours après la greffe, afin d'évaluer la survie neuronale après greffe

### RESULTATS

### Résultats des études comportementales

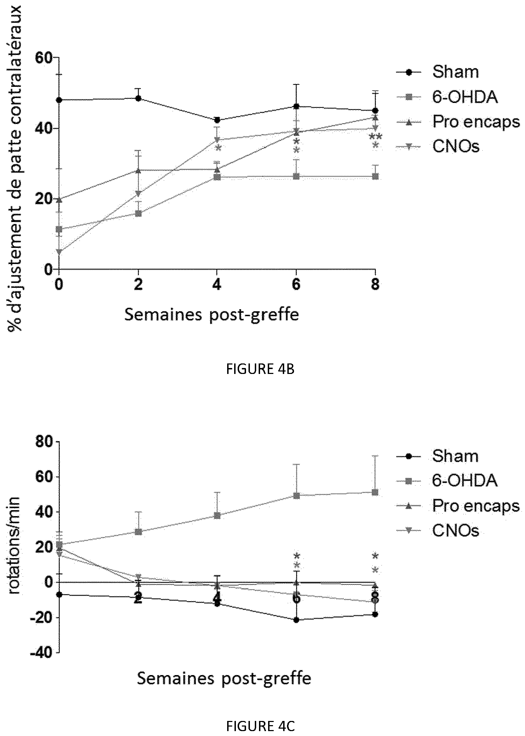

Les études comportementales démontrent un effet très positif des greffes selon l'invention chez les rats greffés (6Pro-encaps et CNOs) puisqu'une correction comportementale est obtenue en 4 à 6 semaines pour le test de stepping Figure 4B) et le test du cylindre (Figure 4A), et en 2 semaines pour le test du rotomètre (Figure 4C), alors que les quelques effets démontrés dans la littérature avec des greffes de progéniteurs montrent une amélioration comportementale après 20 semaines environ (Kriks et al. 2011, Grealish et al. 2014, Kirkeby et al. 2017, Doi et al. 2014).

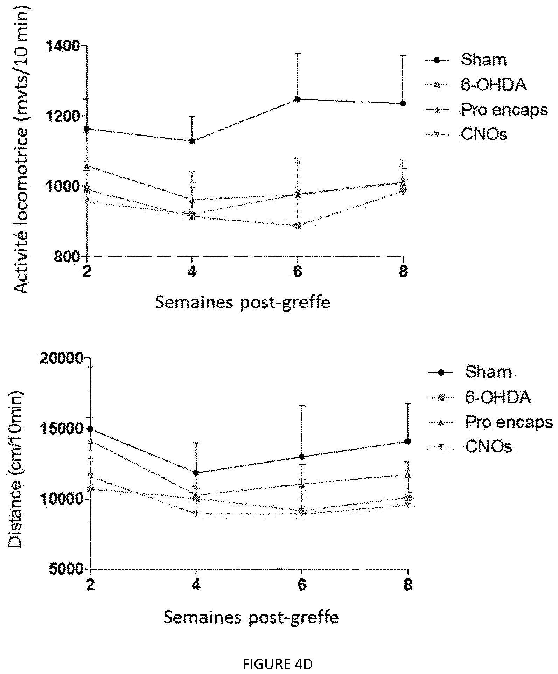

Comme attendu, les greffes selon l'invention n'ont en revanche aucun effet au niveau des résultats d'anxiété (Figure 4D).

### Résultats histologiques

L'analyse histologique des greffes effectuées dans les deux groupes CNO et Pro-encaps montre la présence de 10 à 15% de neurones TH dans les greffes. A titre de comparaison, les études précédemment publiées décrivent des taux de neurones TH après greffe de progéniteurs de l'ordre de 5% (Kirkeby et al. 2017) ou 6% (Kriks et al. 2011).

L'analyse de la survie neuronale montre qu'après 6 jours de greffe on observe des projections axonales pluri-millimétriques dans le cerveau des rats CNOs (figure 3), ce qui confirme la survie importante des neurones après une greffe selon l'invention.

## Revendications

1. Unité de tissu neural formant un amas de cellules neurales pour son utilisation en thérapie cellulaire pour une implantation dans le système nerveux d'un mammifère humain ou non humain, ladite unité de tissu neural étant capable de projeter des axones au sein du tissu hôte, dans laquelle ladite unité de tissu neural contient des cellules neuronales post-mitotiques différenciées immuno-compatibles avec le mammifère destiné à recevoir l'unité de tissu neural et dont le phénotype correspond au phénotype des cellules défaillantes à remplacer, organisées en réseau trois dimensions et noyées dans une couche de matrice extracellulaire, ladite unité étant obtenue à partir d'un microcompartiment cellulaire comprenant une capsule en hydrogel entourant une unique unité de tissu neural, et ladite capsule en hydrogel étant au moins partiellement éliminée avant utilisation de ladite unité de tissu neural.

2. Unité de tissu neural pour son utilisation selon la revendication 1, dans laquelle ladite unité de tissu neural est dépourvue d'hydrogel.

3. Unité de tissu neural pour son utilisation selon la revendication 1 ou 2, dans laquelle les cellules neurales présentent de manière préférée une plus petite dimension comprise entre 50 µm et 500 µm plus ou moins 10%, préférentiellement entre 150 µm et 400 µm plus ou moins 10%, encore plus préférentiellement entre 200 µm et 300 µm plus ou moins 10%.

4. Unité de tissu neural pour son utilisation selon l'une des revendications précédentes, ladite unité de cellules neurales comprenant des cellules neuronales et des cellules gliales.

5. Unité de tissu neural pour son utilisation selon l'une des revendications précédentes, dans laquelle ladite unité comprend entre 10 et 100% de cellules neuronales post-mitotiques dont le phénotype correspond au phénotype des cellules défaillantes à remplacer, préférentiellement entre 50 et 100%, plus préférentiellement plus de 90%.

6. Unité de tissu neural pour son utilisation selon l'une des revendications précédentes, dans laquelle ladite unité contient de 100 à 100.000 cellules neurales.

7. Unité de tissu neural pour son utilisation selon l'une des revendications précédentes, pour le traitement d'une maladie neurodégénérative, telle que la maladie de Parkinson, la maladie de Huntington, la sclérose latérale amyotrophique (ALS), la maladie d'Alzheimer ou les céroïdes-lipofuscinoses neuronales telles que la maladie de Batten.

8. Unité de tissu neural pour son utilisation selon la revendication 7, dans laquelle ladite unité de tissu neural contient des neurones dopaminergiques, pour le traitement de la maladie de Parkinson.

9. Unité de tissu neural pour son utilisation selon la revendication 7, dans laquelle ladite unité de tissu neural contient des neurones GABAergiques, pour le traitement de la maladie de Huntington.

10. Unité de tissu neural pour son utilisation selon l'une des revendications 1 à 9, dans laquelle ladite unité de tissu neural contient des cellules neurales modifiées génétiquement.

11. Unité de tissu neural pour son utilisation selon la revendication 10, pour assister, améliorer ou réparer une fonction de cognition et/ou d'action.

12. Kit d'implantation d'unités de tissu neural comprenant au moins un microcompartiment cellulaire comprenant au moins une capsule d'hydrogel enveloppant une unité de tissu neural selon l'une des revendications 1 à 11, et des moyens d'élimination au moins partielle de la capsule d'hydrogel.

13. Kit d'implantation d'unités de tissu neural selon la revendication 12, comprenant en outre un dispositif d'implantation chirurgical apte à implanter une unité de tissu neural dans le système nerveux d'un mammifère.

14. Kit d'implantation d'unités de tissu neural comprenant au moins une unité de tissu neural selon l'une des revendications 1 à 11, préférentiellement entre 1 et 10.000, plus préférentiellement entre 10 et 1.000 unités de tissu neural dans un dispositif d'implantation chirurgical apte à implanter la ou les unités de tissu neural dans le système nerveux d'un mammifère.

## Patentansprüche

1. Nervengewebeeinheit, die eine Ansammlung von Nervenzellen ausbildet, zur Verwendung in einer Zelltherapie für eine Implantation in das Nervensystem eines menschlichen oder nicht menschlichen Säugetiers, wobei die Nervengewebeeinheit fähig ist, Axone innerhalb des Wirtsgewebes zu projizieren, wobei die Nervengewebeeinheit differenzierte post-mitotische Nervenzellen enthält, die mit dem Säugetier immunkompatibel sind, das dafür bestimmt ist, die Nervengewebeeinheit aufzunehmen, und deren Phänotyp dem Phänotyp der zu ersetzenden defekten Zellen entspricht, die zu einem dreidimensionalen Netzwerk organisiert und in eine Schicht aus extrazellulärer Matrix eingebettet sind, wobei die Einheit aus einem Zellmikrokompartiment erhalten wird, umfassend eine Hydrogelkapsel, die eine einzelne Nervengewebeeinheit umgibt, und wobei die Hydrogelkapsel vor der Verwendung der Nervengewebeeinheit mindestens teilweise entfernt wird.

2. Nervengewebeeinheit zur Verwendung nach Anspruch 1, wobei die Nervengewebeeinheit ohne Hydrogel ist.

3. Nervengewebeeinheit zur Verwendung nach Anspruch 1 oder 2, wobei die Nervenzellen auf bevorzugte Weise eine kleinere Abmessung zwischen 50 µm und 500 µm plus oder minus 10 %, bevorzugt zwischen 150 µm und 400 µm plus oder minus 10 %, stärker bevorzugt zwischen 200 µm und 300 µm plus oder minus 10 % aufweisen.

4. Nervengewebeeinheit zur Verwendung nach einem der vorstehenden Ansprüche, die Nervenzelleneinheit umfassend Nervenzellen und Gliazellen.

5. Nervengewebeeinheit zur Verwendung nach einem der vorstehenden Ansprüche, wobei die Einheit zwischen 10 und 100 % post-mitotische Nervenzellen umfasst, deren Phänotyp dem Phänotyp der zu ersetzenden defekten Zellen entspricht, bevorzugt zwischen 50 und 100 %, stärker bevorzugt mehr als 90 %.

6. Nervengewebeeinheit zur Verwendung nach einem der vorstehenden Ansprüche, wobei die Einheit 100 bis 100.000 Nervenzellen enthält.

7. Nervengewebeeinheit zur Verwendung nach einem der vorstehenden Ansprüche für die Behandlung einer neurodegenerativen Krankheit, wie Parkinson-Krankheit, Huntington-Krankheit, amyotrophische Lateralsklerose (ALS), Alzheimer-Krankheit oder neuronale Zeroidlipofuszinosen, wie Batten-Syndrom.

8. Nervengewebeeinheit zur Verwendung nach Anspruch 7, wobei die Nervengewebeeinheit dopaminerge Neuronen enthält, für die Behandlung der Parkinson-Krankheit.

9. Nervengewebeeinheit zur Verwendung nach Anspruch 7, wobei die Nervengewebeeinheit GABAerge Neuronen enthält, für die Behandlung der Huntington-Krankheit.

10. Nervengewebeeinheit zur Verwendung nach einem der Ansprüche 1 bis 9, wobei die Nervengewebeeinheit genetisch modifizierte Nervenzellen enthält.

11. Nervengewebeeinheit zur Verwendung nach Anspruch 10, zum Unterstützen, Verbessern oder Korrigieren einer kognitiven und/oder Handlungsfunktion.

12. Kit zum Implantieren von Nervengewebeeinheiten, umfassend mindestens ein zelluläres Mikrokompartiment, umfassend mindestens eine Hydrogelkapsel, die eine Nervengewebeeinheit nach einem der Ansprüche 1 bis 11 umhüllt, und Mittel zum mindestens teilweisen Entfernen der Hydrogelkapsel.

13. Kit zum Implantieren von Nervengewebeeinheiten nach Anspruch 12, ferner umfassend eine chirurgische Implantationsvorrichtung, die geeignet ist, um eine Nervengewebeeinheit in das Nervensystem eines Säugetiers zu implantieren.

14. Kit zum Implantieren von Nervengewebeeinheiten, umfassend mindestens eine Nervengewebeeinheit nach einem der Ansprüche 1 bis 11, vorzugsweise zwischen 1 und 10.000, besonders bevorzugt zwischen 10 und 1.000 Nervengewebeeinheiten, in einer chirurgischen Implantationsvorrichtung, die geeignet ist, um die Nervengewebeeinheit(en) in das Nervensystem eines Säugetiers zu implantieren.

## Claims

1. Neural tissue unit forming a neural cell cluster for use in cell therapy for implantation in the nervous system of a human or non-human mammal, the neural tissue unit being capable of projecting axons within the host tissue, wherein said neural tissue unit contains differentiated post-mitotic neuronal cells which are immuno-compatible with the mammal intended to receive the neural tissue unit and of which the phenotype corresponds to the phenotype of the defective cells to be replaced, and which are organized into a three-dimensional array and embedded in an extracellular matrix layer, said unit being obtained from a cellular microcompartment comprising a hydrogel capsule surrounding a single neural tissue unit, and said hydrogel capsule being at least partially removed prior to use of said neural tissue unit.

2. Neural tissue unit for use according to claim 1, wherein said neural tissue unit is free of hydrogel.

3. Neural tissue unit for use according to either claim 1 or claim 2, wherein the neural cells preferably have a smaller dimension of between 50 µm and 500 µm plus or minus 10%, preferably between 150 µm and 400 µm plus or minus 10%, even more preferably between 200 µm and 300 µm plus or minus 10%.

4. Neural tissue unit for use according to any of the preceding claims, said neural cell unit comprising neuronal cells and glial cells.

5. Neural tissue unit for use according to any of the preceding claims, wherein said unit comprises between 10 and 100% post-mitotic neuronal cells of which the phenotype corresponds to the phenotype of the defective cells to be replaced, preferably between 50 and 100%, more preferably more than 90%.

6. Neural tissue unit for use according to any of the preceding claims, wherein said unit contains 100 to 100,000 neural cells.

7. Neural tissue unit for use according to any of the preceding claims for treating a neurodegenerative disease, such as Parkinson's disease, Huntington's disease, amyotrophic lateral sclerosis (ALS), Alzheimer's disease or neuronal ceroid lipofuscinosis, such as Batten disease.

8. Neural tissue unit for use according to claim 7, wherein said neural tissue unit contains dopaminergic neurons for treating Parkinson's disease.

9. Neural tissue unit for use according to claim 7, wherein said neural tissue unit contains GABAergic neurons for treating Huntington's disease.

10. Neural tissue unit for use according to any of claims 1 to 9, wherein said neural tissue unit contains genetically modified neural cells.

11. Neural tissue unit for use according to claim 10 to assist, improve or repair a cognition and/or action function.

12. Kit for implanting neural tissue units comprising at least one cell microcompartment comprising at least one hydrogel capsule enclosing a neural tissue unit according to any of claims 1 to 11, and means for at least partially removing the hydrogel capsule.

13. Kit for implanting neural tissue units according to claim 12, further comprising a surgical implantation device capable of implanting a neural tissue unit in the nervous system of a mammal.

14. Kit for implanting neural tissue units comprising at least one neural tissue unit according to any of claims 1 to 11, preferably between 1 and 10,000, more preferably between 10 and 1,000 units of neural tissue in a surgical implantation device capable of implanting the neural tissue unit(s) in the nervous system of a mammal.
